# EUROPÄISCHE PATENTSCHRIFT

(11) **EP 1 132 367 B1**
(45) Veröffentlichungstag und Bekanntmachung des Hinweises auf die Patenterteilung: **18.06.2003**
(21) Anmeldenummer: 01710004.1
(22) Anmeldetag: 01.02.2001
(51) Int. Cl.: C07C 46/08, C07C 50/02

(54) **Verfahren zur Herstellung von 2,3,5-Trimethyl-p-benzochinon**
Process for the preparation of 2,3,5-trimethyl-p-benzoquinone
Procédé de préparation de la 2,3,5-triméthyl-p-benzoquinone

(30) Priorität: 09.03.2000 DE 10011405
(43) Veröffentlichungstag der Anmeldung: 12.09.2001
(73) Patentinhaber: Degussa-Hüls Aktiengesellschaft, 60287 Frankfurt am Main (DE)
(72) Erfinder: Maassen, Ralf, Dr., 63457 Hanau (DE); Krill, Steffen, Dr., 67346 Speyer (DE); Huthmacher, Klaus, Dr., 63584 Geinhausen (DE)

(56) Entgegenhaltungen:
- EP-A- 0 387 820
- SHIMIZU M ET AL.: "Synthesis of Alkyl Substituted p-Benzoquinones from the Corresponding Phenols Using Molecular Oxygen Catalyzed by Copper(II) Chloride-Amine Hydrochloride Systems" BULLETIN OF THE CHEMICAL SOCIETY OF JAPAN, Bd. 65, Nr. 6, 1992, Seiten 1522-1526, XP001005330

## Beschreibung

Die vorliegende Erfindung betrifft ein neues Verfahren zur Herstellung von 2,3,5-Trimethyl-p-benzochinon durch Oxidation von 2,3,5- oder 2,3,6-Trimethylphenol mittels Sauerstoff oder eines Sauerstoff enthaltenden Gasgemisches in Gegenwart eines zweiphasigen flüssigen Reaktionsmediums bestehend aus Wasser und einer Neocarbonsäure mit 8 bis 11 C-Atomen mit einem Kupfer(II)halogenid enthaltenden Katalysatorsystem bei erhöhter Temperatur.

2,3,5-Trimethyl-p-benzochinon ist ein Zwischenprodukt, das unter anderem für die Herstellung von α-Tocopherolen (Vitamin E) verwendet wird.

Die Oxidation von Trimethylphenolen zu 2,3,5-Trimethyl-p-benzochinon ist bekannt.

Von den vielen beschriebenen Verfahren ist die Oxidation mittels Sauerstoff oder eines Sauerstoff enthaltenden Gasgemisches unter Katalyse durch Kupfersalz-haltige Katalysatorsysteme in zweiphasigen flüssigen Reaktionsmedien von besonderem technischen Interesse. Der Vorteil dieser Verfahren liegt neben den ausgezeichneten erreichbaren Ausbeuten und Selektivitäten vor allem in der Verwendung eines kostengünstigen und einfach herzustellenden Katalysatorsystems begründet, das in wäßriger Phase vorliegt und somit nach der Reaktion durch einfache Phasentrennung von der das Produkt enthaltenden organischen Phase abgetrennt und unter minimalem Aufwand und praktisch ohne Aktivitäts- und Selektivitätsverlust recycliert werden kann.

Nach der EP 0 127 888 gelingt die Oxidation von Trimethylphenol zu Trimethyl-p-benzochinon in guten Ausbeuten mittels molekularem Sauerstoff in Gegenwart eines separat hergestellten Alkalimetall- oder Ammonium-Halogenocuprats der Kupfer-Oxidationsstufe +2, eventuell unter Zusatz eines Alkalimetall- oder Ammoniumhalogenids. Als Reaktionsmedium wird hier ein Gemisch aus Wasser und einem aliphatischen Alkohol mit vier bis zehn Kohlenstoffatomen beschrieben. Entsprechend der EP 0 167 153 läßt sich die Ausbeute an Trimethyl-p-benzochinon noch steigern und die Nebenproduktbildung weiter vermindern, wenn dem beschriebenen Alkalimetall- oder Ammonium-Halogenocuprat zusätzlich katalytische Mengen an Kupfer(I)hydroxid und/oder Kupfer(I)chlorid zugefügt werden und die alkoholische Trimethylphenol-Lösung langsam zu der wäßrigen Katalysatorlösung zudosiert wird.

Die EP 0 294 584 beschreibt ein Verfahren zur Oxidation von Trimethylphenol zu Trimethyl-p-benzochinon durch molekularen Sauerstoff unter Verwendung einer wäßrigen Lösung von Kupfer(II)chlorid und Lithiumchlorid als Katalysator. Als Lösungsmittel für das Edukt und somit als zweite flüssige Phase wird ein Gemisch aus einem aromatischen Kohlenwasserstoff, vorzugsweise Benzol, Toluol, Xylol oder Chlorbenzol, und einem niederen aliphatischen Alkohol mit eins bis vier Kohlenstoffatomen verwendet.

Nach der EP 0 475 272 können anstelle von Lithiumchlorid auch Erdalkalihalogenide als wäßrige Lösung in Kombination mit Kupfer(II)chlorid die beschriebene Reaktion effizient katalysieren. Als organisches Lösungsmittel kommen hierbei sowohl aliphatische Alkohole mit fünf bis zehn Kohlenstoffatomen, als auch Gemische aus aromatischen Kohlenwasserstoffen und aliphatischen Alkoholen mit eins bis vier Kohlenstoffatomen in Betracht.

In der EP 0 369 823 wird die Oxidation von Trimethylphenol zu Trimethyl-p-benzochinon unter Verwendung eines Katalysatorsystems beschrieben, welches neben Kupfer(II)chlorid zusätzlich ein Salz eines Hydroxylamins, Oxims oder Amins mit einer anorganischen Säure oder ein freies Oxim enthält. Als organische Phase dienen bei diesem Verfahren entweder aliphatische Alkohole mit vier bis zehn Kohlenstoffatomen, oder auch Gemische aus aromatischen Kohlenwasserstoffen und aliphatischen Alkoholen mit eins bis sechs Kohlenstoffatomen.

Nachteilig bei allen beschriebenen Verfahren ist die Reaktionsführung bei Temperaturen oberhalb oder nur knapp unterhalb des Flammpunktes der eingesetzten Lösungsmittel. Die damit verbundene Explosionsgefahr birgt enorme Risiken für die technische Umsetzung der Prozesse, vor allem, da wegen der Notwendigkeit des Vorhandenseins molekularen Sauerstoffs als Oxidationsmittel eine Inertisierung des Reaktionsgemisches, wie sie sonst bei Arbeiten in der Nähe oder oberhalb des Flammpunktes des verwendeten Lösungsmittels üblich ist, nicht realisiert werden kann. Für die beschriebene Reaktion ist daher ein genügender Sicherheitsabstand zwischen Reaktionstemperatur und Flammpunkt der organischen Bestandteile dringend erforderlich, damit auch bei Temperaturanstiegen durch kurzzeitigen unkontrollierten Verlauf der Reaktion oder bei technischen Anlagenproblemen eine sichere Fahrweise der Anlage garantiert werden kann. Bei den beschriebenen Verfahren ist dies durchweg nicht der Fall. Bei den jeweils bevorzugten Reaktionstemperaturen von 60°C oder höher, die zum Erreichen guter Ausbeuten erforderlich sind, werden die Flammpunkte der beschriebenen organischen Lösungsmittel entweder überschritten oder nur knapp unterschritten (vgl. Tabelle 1).

**Tabelle 1**

| Lösungsmittel | Flammpunkt [°C] |
|---|---|
| Methanol | 11 |
| Ethanol | 12 |
| 1-Propanol | 15 |
| 1-Butanol | 30 |
| 1-Pentanol | 47 |
| 1-Hexanol | 60 |
| 1-Heptanol | 73 |
| 1-Octanol | 81 |
| 1-Nonanol | 75 |
| 1-Decanol | 82 |
| Benzol | -11 |
| Toluol | 6 |
| p-Xylol | 25 |
| Chlorbenzol | 28 |

In der EP 0 387 820 wird erstmals auf dieses Problem der sicherheitstechnisch bedenklichen Reaktionsführung eingegangen. Als Lösung des Problems wird die Verwendung von aliphatischen Alkoholen mit zwölf bis achtzehn Kohlenstoffatomen und Flammpunkten oberhalb 120°C als organisches Lösungsmittel unter Einsatz von Kupfer(II)halogenid in Kombination mit Alkali- oder Erdalkalihalogeniden in Form einer wäßrigen Lösung als Katalysator der Umsetzung beschrieben. Bei den bevorzugten Reaktionstemperaturen von 80°C bis 90°C wird so bei leicht verminderten Ausbeuten an Trimethyl-p-benzochinon die Explosionsgefahr der Reaktionsmischung sicher vermieden. Ein weiterer Vorteil der verwendeten langkettigen Alkohole ist in ihrem hohen Siedepunkt zu sehen, der deutlich oberhalb dem des Trimethyl-pbenzochinons liegt. Dadurch kann das Reaktionsprodukt auf einfache Weise nach Phasentrennung destillativ als Leichtsieder aus dem rohen Produktgemisch isoliert werden. Ein Nachteil der verwendeten Alkohole mit zwölf bis achtzehn Kohlenstoffatomen ist jedoch ihr relativ hoher Schmelzpunkt (Tabelle 2). So liegen diese Verbindungen bei Raumtemperatur als wachsartige Feststoffe vor, was in Bezug auf eine technische Realisierung des Verfahrens einige Probleme mit sich bringt. So muß das Lösungsmittel vor Beginn der Reaktion zunächst aufgeschmolzen werden, was einen zusätzlichen Verfahrensschritt und Energieaufwand bedeutet. Zudem muß unter nicht unerheblichem Aufwand dafür Sorge getragen werden, daß zu jeder Zeit, auch im Falle technischer Störungen, alle Anlagenteile auf einer Temperatur oberhalb des Schmelzpunktes des Alkohols gehalten werden, da ansonsten die Gefahr des Erstarrens der organischen Phase in der Anlage und somit die Verstopfung von Anlagenteilen droht.

**Tabelle 2**

| Lösungsmittel | Flammpunkt [°C] | Schmelzpunkt [°C] |
|---|---|---|
| 1-Dodecanol | 127 | 22-24 |
| 1-Tetradecanol | 141 | 37-39 |
| 1-Hexadecanol | 135 | 49 |
| 1-Octadecanol | 192 | 55-58 |

Die Aufgabe der vorliegenden Erfindung war es daher, auf der Basis des Stands der Technik ein Verfahren bereitzustellen, welches die Oxidation von Trimethylphenol zu Trimethyl-p-benzochinon in guten Ausbeuten und unter sicherem Ausschluß der Explosionsgefahr der Reaktionsmischung ermöglicht und gleichzeitig die in der Beurteilung des Stands der Technik aufgeführten Nachteile der bestehenden Verfahren vermeidet.

Es wurde nun gefunden, daß sich die Aufgabe lösen läßt, wenn man als Lösungsmittelsystem ein Gemisch aus Wasser und einer Neocarbonsäure mit 8 bis 11 C-Atomen, vorzugsweise Neodecansäure einsetzt, insbesondere wenn als Katalysatoren Kupfer(II)halogenide verwendet werden, denen zur Aktivitätssteigerung Erdalkali-, Alkali- oder Übergangsmetallhalogenide oder Halogenide eines Elements der seltenen Erden zugesetzt werden.

Neodecansäure bezeichnet ein Gemisch aus Octan-, Nonan- und Decansäure (Hersteller Firma Exxon Chemical).

Dieses Ergebnis war insofern überraschend, als daß Neodecansäure mit < 0,01 Gew.-% eine sehr geringe Löslichkeit in Wasser aufweist und somit eine schlechte Wechselwirkung der wäßrigen Katalysatorphase mit der organischen Substratphase und damit auch eine geringe Eignung der Neodecansäure für die Oxidation im Zweiphasensystem zu erwarten war.

Es zeigte sich jedoch, daß die Oxidation von Trimethylphenol zu Trimethyl-p-benzochinon in Gegenwart eines Reaktionsmediums bestehend aus Wasser und Neodecansäure und unter Katalyse durch ein mindestens Kupfer(II)halogenid enthaltendes Katalysatorsystem sehr vorteilhaft verläuft. Bei den bevorzugten Reaktionstemperaturen von 50°C bis 100°C, vorzugsweise von 60°C bis 90°C, wird der Flammpunkt des Lösungsmittels von 122°C deutlich unterschritten, so daß eine Durchführung der Oxidation unter Ausschluß von Explosionsgefahren sicher gewährleistet werden kann.

Durch die geringe Wasserlöslichkeit der Neodecansäure kann nach erfolgter Reaktion die wäßrige Katalysatorphase in einfacher Weise durch Phasentrennung von der organischen, das Produkt enthaltenden Phase abgetrennt werden. Die Katalysatorphase kann so unter minimalem Aufwand recycliert und mehrfach ohne signifikanten Aktivitäts- oder Selektivitätsverlust wiederverwendet werden.

Der im Vergleich zum Siedepunkt des Trimethyl-pbenzochinons (198°C) relativ hohe Siedepunkt der Neodecansäure (243°C bis 253°C) erlaubt zudem eine einfache und schonende destillative Isolierung des thermisch empfindlichen Produkts als Leichtsieder aus dem rohen Produktgemisch und ermöglicht eine einfache Rückführung der Neocarbonsäure in die Reaktion.

Der besondere Vorteil der Neodecansäure im Vergleich zu den nach dem Stand der Technik bekannten längerkettigen Alkoholen mit 12 bis 18 Kohlenstoffatomen liegt jedoch in dem sehr niedrigen Schmelzpunkt von -39°C begründet, wodurch die in der Beurteilung des Stands der Technik beschriebenen, zum Teil gravierenden Nachteile bezüglich des Aufschmelzen des Lösungsmittels und der Vermeidung der Gefahr der Verstopfung von Anlagenteilen umgangen werden.

Gegenstand der Erfindung ist daher ein Verfahren zur Herstellung von 2,3,5-Trimethyl-p-benzochinon durch Oxidation von 2,3,5- oder 2,3,6-Trimethylphenol mittels Sauerstoff oder eines Sauerstoff enthaltenden Gases in einem zweiphasigen flüssigen Reaktionsmedium unter Einsatz eines mindestens Kupfer(II)halogenid enthaltenden Katalysators bei erhöhter Temperatur, dadurch gekennzeichnet, daß man die Umsetzung in einem Gemisch bestehend aus Wasser und Neodecansäure und bei Temperaturen zwischen 50°C und 100°C durchführt.

In dem erfindungsgemäßen Verfahren können zur Steigerung der Katalysatoraktivität dem Kupfer(II)halogenid eine oder mehrere Verbindungen aus der Klasse der Alkali-, Erdalkalioder Übergangsmetallhalogenide oder der Halogenide der Elemente der seltenen Erden zugesetzt werden.

Als geeignete Kupfer(II)halogenide seien im wesentlichen Kupfer(II)chlorid und Kupfer(II)bromid genannt. Bei den zur Aktivitätssteigerung zugesetzten Alkali-, Erdalkali- und Übergangsmetallhalogeniden sowie Halogeniden eines Elements der seltenen Erden handelt es sich bevorzugt um Lithiumchlorid, Natriumchlorid, Magnesiumchlorid, Calciumchlorid, Chrom(III)chlorid und Cer(III)chlorid.

Die Darstellung der wäßrigen Katalysatorphase erfolgt durch einfache Mischung der wäßrigen Lösungen der Einzelkomponenten oder durch Auflösen der festen Salzverbindungen in Wasser, was die Prozeßführung deutlich vereinfacht.

Das molare Verhältnis des Kupfer(II)halogenids bezüglich Trimethylphenol ist in weiten Grenzen variierbar und beträgt üblicherweise Kupfersalz/Trimethylphenol = 0,1 - 10, bevorzugt 0,2 - 3.

Die zur Aktivitätssteigerung des Katalysators zugesetzten Halogenide können bezogen auf das Trimethylphenol in der 0,1 bis 12fachen molaren Menge eingesetzt werden, bevorzugt wird eine 0,2 bis 8fache molare Menge. Die Konzentration des Kupferhalogenids in der wäßrigen Katalysatorphase kann zwischen 1 Gew.-% - 70 Gew.-% variiert werden, vorzugsweise werden Konzentrationen zwischen 5 Gew.-% - 30 Gew.-% verwendet, die aktivitätssteigernden Halogenide werden bevorzugt in einem Konzentrationsbereich zwischen 5 Gew.-% - 80 Gew.-% verwendet.

Als zusätzliche Aktivatoren der Umsetzung können die aus dem Stand der Technik bekannten Systeme eingesetzt werden, am vorteilhaftesten werden Kupfersalze wie Kupfer(I)chlorid oder das entsprechende Hydroxid eingesetzt.

Als Oxidationsmittel im erfindungsgemäßen Verfahren dient Sauerstoff in reiner Form oder in verdünnter Form, z.B. Luft. Bezogen auf 1 L Reaktionsgemisch werden dabei in der Regel pro Stunde 10 bis 150 L gasförmiger Sauerstoff zugeführt. Das neue Verfahren wird in der Regel bei Normaldruck durchgeführt. Das Verfahren kann ebenfalls unter Druck durchgeführt werden; eine Druckfahrweise ist insbesondere bei sauerstoffhaltigen Gasgemischen angebracht. Es kann sowohl in kontinuierlicher als auch diskontinuierlicher Arbeitsweise vorgenommen werden.

Zur Durchführung der Reaktion wird Trimethylphenol in Neodecansäure gelöst und zur wäßrigen, den Katalysator enthaltenden Phase zudosiert. In einer anderen Ausführungsform wird ein Teil des organischen Lösungsmittels vor Reaktionsbeginn mit der wäßrigen Phase vorgelegt und die Trimethylphenol-Lösung zudosiert. In wiederum einer anderen Variante der Reaktionsführung wird die Reaktion batchweise durchgeführt, indem alle Komponenten unter Rühren vorgelegt werden und anschließend mit der Zudosierung des sauerstoffhaltigen Gases begonnen wird.

Die Konzentration an Trimethylphenol in der organischen Phase kann in weiten Konzentrationsbereichen variiert werden, im allgemeinen werden Trimethylphenol-Konzentrationen zwischen 5 Gew.-% und 80 Gew.-% eingestellt, bevorzugt Konzentrationen zwischen 10 Gew.-% und 50 Gew.-%.

Das Volumenverhältnis von Wasser zu organischem Lösungsmittel kann in einem Bereich zwischen 10:1 und 1:10 schwanken, bevorzugt wird ein Bereich zwischen 3:1 und 1:5.

Die Reaktionstemperatur kann über ein weites Temperaturintervall variiert werden, bevorzugt wird die Umsetzung zwischen 50°C und 100°C durchgeführt, in einer besonders bevorzugten Ausführungsform wird zwischen 60°C und 90°C gearbeitet.

Das Reaktionsprodukt 2,3,5-Trimethyl-p-benzochinon kann auf üblichem Weg, zum Beispiel mittels Vakuum- oder Wasserdampfdestillation isoliert werden.

Das erfindungsgemäße Verfahren ist einfach durchzuführen und liefert das Reaktionsprodukt in guter Ausbeute und hoher Reinheit.

Gemäß Beispiel 16 kann die zurückgewonnene Katalysatorlösung mehrmals wieder eingesetzt werden, ohne daß eine Ausbeuteminderung zu verzeichnen ist.

Die Ausbeutebestimmungen wurden auf einem HPLC-System der Firma Jasco durchgeführt, bestehend aus einem UV-Detektor UV 975, einer Pumpe PU 980 und einem Autosampler AS 950. Die verwendete Säule war eine Inertsil-ODS 3V-5µ, 250 x 4,6 mm Innendurchmesser der Firma GL Sciences Inc.. Als externer Standard diente Trimethyl-p-benzochinon, welches durch Destillation und mehrfache Kristallisation gereinigt wurde.

Die folgenden Beispiele sollen die Erfindung näher erläutern.

TMP steht für 2,3,6-Trimethylphenol. TMQ steht für 2,3,5-Trimethyl-p-benzochinon.

### Beispiel 1 bis 15

In einem Glasreaktor wurden Kupfer(II)chlorid und ein Alkali-, Erdalkali- oder Übergangsmetallhalogenid in den aus Tabelle 3 ersichtlichen Mengen als gesättigte wäßrige Lösung vorgelegt, mit der jeweils angegebenen Menge Neodecansäure versetzt und auf die in Tabelle 3 dargestellte Reaktionstemperatur aufgeheizt. Anschließend wurde unter Rühren (800 Upm) und Sauerstoffbegasung über eine Fritte innerhalb von 3h eine Lösung aus 24 g TMP (176 mmol) in 120 mL Neodecansäure zugetropft (Beispiel 7: sofortige Zugabe der gesamten Menge; batch-Ansatz). Nach Beendigung der Zugabe wurde noch weitere 3 h (Beispiel 1: 4h; Beispiel 14: 5h) bei der angegebenen Temperatur unter Sauerstoffbegasung nachgerührt und der Reaktionsverlauf per HPLC verfolgt. Nach Ablauf der Reaktion wurden die Phasen getrennt, die organische Phase 2mal mit gesättigter Natriumchloridlösung gewaschen und die TMQ-Ausbeute durch HPLC mit externem Standard ermittelt.

**Tabelle 3**

| **Beispiel** | **Katalysator (Molmenge [mmol])** | **Stöchiometrie TMP/CuCl**_{**2**}**/Halogenid** | **Temperatur [°C]** | **vorgelegte Menge Neode- cansäure [mL]** | **TMQ- Ausbeute [%]** |
|---|---|---|---|---|---|
| 1 | CuCl₂ (176) LiCl (704) | 1:1:4 | 60 | 0 | 88,9 |
| 2 | CuCl₂ (132) LiCl (528) | 1:0,75:3 | 70 | 0 | 89,5 |
| 3 | CuCl₂ (176) LiCl (704) | 1:1:4 | 70 | 0 | 90,9 |
| 4 | CuCl₂ (176) LiCl (704) | 1:1:4 | 70 | 30 | 91,2 |
| 5 | CuCl₂ (88) LiCl (352) | 1:0,5:2 | 80 | 0 | 84,8 |
| 6 | CuCl₂ (132) LiCl (528) | 1:0,75:3 | 80 | 0 | 87,3 |
| 7 | CuCl₂ (132) LiCl (528) | 1:0,75:3 | 80 | 0 | 87,2 |
| 8 | CuCl₂ (176) LiCl (704) | 1:1:4 | 80 | 0 | 90,2 |
| 9 | CuCl₂ (132) LiCl (528) | 1:0,75:3 | 90 | 0 | 86,4 |
| 10 | CuCl₂ (132) LiCl (528) | 1:0,75:3 | 90 | 30 | 87,4 |
| 11 | CuCl₂ (132) LiCl (528) | 1:0,75:3 | 90 | 60 | 87,3 |
| 12 | CuCl₂ (176) LiCl (704) | 1:1:4 | 90 | 0 | 89,8 |
| 13 | CuCl₂ (176) MgCl₂ (352) | 1:1:2 | 90 | 0 | 89,0 |
| 14 | CuCl₂ (176) CrCl₃ (352) | 1:1:2 | 90 | 0 | 92,3 |
| 15 | CuCl₂ (132) LiCl (528) | 1:0,75:3 | 100 | 0 | 88,5 |

### Beispiel 16

Die Durchführung der Reaktion gemäß Ausführungsbeispiel 3 wurde wiederholt, wobei die nach Phasentrennung erhaltene Katalysatorlösung durch Einengen am Rotationsverdampfer vom zusätzlich durch die Oxidationsreaktion erzeugten Reaktionswasser befreit wurde. Die so erhaltene wäßrige Katalysatorlösung wurde ohne weitere Behandlung direkt wieder in die Reaktion eingesetzt und das Verfahren insgesamt sechsmal in Folge ausgeführt. Die so nach der fünften Wiederverwendung der ursprünglichen Katalysatorlösung erhaltene TMQ-Ausbeute betrug noch 89,7 %.

## Patentansprüche

1. Verfahren zur Herstellung von 2,3,5-Trimethyl-p-benzochinon durch Oxidation von 2,3,5- oder 2,3,6-Trimethylphenol mittels Sauerstoff oder eines Sauerstoff enthaltenden Gases in einem zweiphasigen flüssigen Reaktionsmedium unter Einsatz eines mindestens Kupfer(II)halogenid enthaltenden Katalysators bei Temperaturen zwischen 50°C und 100°C, **dadurch gekennzeichnet, daß** man die Umsetzung in einem Gemisch bestehend aus Wasser und einer Neocarbonsäure mit 8 bis 11 C-Atomen durchführt.

2. Verfahren zur Herstellung von 2,3,5-Trimethyl-p-benzochinon gemäß Anspruch 1, **dadurch gekennzeichnet, daß** man als Neocarbonsäure Neodecansäure und deren Isomeren verwendet.

3. Verfahren zur Herstellung von 2,3,5-Trimethyl-p-benzochinon gemäß Anspruch 1, **dadurch gekennzeichnet, daß** man die Umsetzung in Gegenwart von einem Kupfer(II)halogenid und einem Alkalihalogenid als Katalysatorsystem durchführt.

4. Verfahren zur Herstellung von 2,3,5-Trimethyl-p-benzochinon gemäß Anspruch 3, **dadurch gekennzeichnet, daß** man als Alkalihalogenid Lithiumchlorid oder Natriumchlorid verwendet.

5. Verfahren zur Herstellung von 2,3,5-Trimethyl-p-benzochinon gemäß Anspruch 1, **dadurch gekennzeichnet, daß** man die Umsetzung in Gegenwart von einem Kupfer(II)halogenid und einem Erdalkalihalogenid als Katalysatorsystem durchführt.

6. Verfahren zur Herstellung von 2,3,5-Trimethyl-p-benzochinon gemäß Anspruch 5, **dadurch gekennzeichnet, daß** man als Erdalkalihalogenid Magnesiumchlorid oder Calciumchlorid verwendet.

7. Verfahren zur Herstellung von 2,3,5-Trimethyl-p-benzochinon gemäß Anspruch 1, **dadurch gekennzeichnet, daß** man die Umsetzung in Gegenwart von einem Kupfer(II)halogenid und einem Übergangsmetallhalogenid als Katalysatorsystem durchführt.

8. Verfahren zur Herstellung von 2,3,5-Trimethyl-p-benzochinon gemäß Anspruch 7, **dadurch gekennzeichnet, daß** man als Übergangsmetallhalogenid Chrom(III)chlorid verwendet.

9. Verfahren zur Herstellung von 2,3,5-Trimethyl-p-benzochinon gemäß Anspruch 1, **dadurch gekennzeichnet, daß** man die Umsetzung in Gegenwart von einem Kupfer(II)halogenid und einem Halogenid eines Elements der seltenen Erden als Katalysator durchführt.

10. Verfahren zur Herstellung von 2,3,5-Trimethyl-p-benzochinon gemäß Anspruch 9, **dadurch gekennzeichnet, daß** man als Halogenid eines Elements der seltenen Erden Cer(III)chlorid verwendet.

## Claims

1. A process for the preparation of 2,3,5-trimethyl-p-benzoquinone by oxidation of 2,3,5- or 2,3,6-trimethylphenol using oxygen or a gas containing oxygen in a two-phase liquid reaction medium using a catalyst containing at least copper (II) halide at temperatures from 50°C to 100°C, **characterised in that** the reaction is carried out in a mixture composed of water and a neocarboxylic acid having 8 to 11 carbon atoms.

2. A process for the preparation of 2,3,5-trimethyl-p-benzoquinone according to claim 1,
**characterised in that**
the neocarboxylic acid used is neodecanoic acid and isomers thereof.

3. A process for the preparation of 2,3,5-trimethyl-p-benzoquinone according to claim 1,
**characterised in that**
the reaction is carried out in the presence of a copper (II) halide and an alkali halide as catalyst system.

4. A process for the preparation of 2,3,5-trimethyl-p-benzoquinone according to claim 3,
**characterised in that**
the alkali halide used is lithium chloride or sodium chloride.

5. A process for the preparation of 2,3,5-trimethyl-p-benzoquinone according to claim 1,
**characterised in that**
the reaction is carried out in the presence of a copper (II) halide and an alkaline earth halide as catalyst system.

6. A process for the preparation of 2,3,5-trimethyl-p-benzoquinone according to claim 5,
**characterised in that**
the alkaline earth halide used is magnesium chloride or calcium chloride.

7. A process for the preparation of 2,3,5-trimethyl-p-benzoquinone according to claim 1,
**characterised in that**
the reaction is carried out in the presence of a copper(II)halide and a transition metal halide as catalyst system.

8. A process for the preparation of 2,3,5-trimethyl-p-benzoquinone according to claim 7,
**characterised in that**
the transition metal halide used is chromium(III)chloride.

9. A process for the preparation of 2,3,5-trimethyl-p-benzoquinone according to claim 1,
**characterised in that**
the reaction is carried out in the presence of a copper (II) halide and a halide of an element of the rare earths as catalyst.

10. A process for the preparation of 2,3,5-trimethyl-p-benzoquinone according to claim 9,
**characterised in that**
the halide of an element of the rare earths used is cerium (III) chloride.

## Revendications

1. Procédé pour la préparation de 2,3,5-triméthyl-p-benzoquinone par oxydation de 2,3,5-triméthylphénoi ou de 2,3,6-triméthylphénol au moyen d'oxygène ou d'un gaz contenant de l'oxygène dans un milieu réactionnel liquide à deux phases en utilisant un catalyseur contenant au moins un halogénure de cuivre (II) à des températures entre 50°C et 100°C, **caractérisé en ce qu'**on réalise la transformation dans un mélange constitué d'eau et d'un acide néocarboxylique comprenant 8 à 11 atomes de carbone.

2. Procédé pour la préparation de 2,3,5-triméthyl-p-benzoquinone selon la revendication 1, **caractérisé en ce qu'**on utilise de l'acide néodécanoique et ses isomères comme acide néocarboxylique.

3. Procédé pour la préparation de 2,3,5-triméthyl-p-benzoquinone selon la revendication 1, **caractérisé en ce qu'**on effectue la transformation en présence d'un halogénure de cuivre (II) et d'un halogénure de métal alcalin comme système de catalyseur.

4. Procédé pour la préparation de 2,3,5-triméthyl-p-benzoquinone selon la revendication 3, **caractérisé en ce qu'**on utilise du chlorure de lithium ou du chlorure de sodium comme halogénure de métal alcalin.

5. Procédé pour la préparation de 2,3,5-triméthyl-p-benzoquinone selon la revendication 1, **caractérisé en ce qu'**on effectue la transformation en présence d'un halogénure de cuivre (II) et d'un halogénure de métal alcalino-terreux comme système de catalyseur.

6. Procédé pour la préparation de 2,3,5-triméthyl-p-benzoquinone selon la revendication 5, **caractérisé en ce qu'**on utilise du chlorure de magnésium ou du chlorure de calcium comme halogénure de métal alcalino-terreux.

7. Procédé pour la préparation de 2,3,5-triméthyl-p-benzoquinone selon la revendication 1, **caractérisé en ce qu'**on effectue la transformation en présence d'un halogénure de cuivre (II) et d'un halogénure d'un métal de transition comme système de catalyseur.

8. Procédé pour la préparation de 2,3,5-triméthyl-p-benzoquinone selon la revendication 7, **caractérisé en ce qu'**on utilise du chlorure de chrome (III) comme halogénure de métal de transition.

9. Procédé pour la préparation de 2,3,5-triméthyl-p-benzoquinone selon la revendication 1, **caractérisé en ce qu'**on effectue la transformation en présence d'un halogénure de cuivre (II) et d'un halogénure d'un élément des terres rares comme catalyseur.

10. Procédé pour la préparation de 2,3,5-triméthyl-p-benzoquinone selon la revendication 9, **caractérisé en ce qu'**on utilise du chlorure de cérium (III) comme halogénure d'un élément des terres rares.
